# EUROPÄISCHE PATENTSCHRIFT

(11) **EP 2 262 556 B1**
(45) Veröffentlichungstag und Bekanntmachung des Hinweises auf die Patenterteilung: **29.05.2013**
(21) Anmeldenummer: 09715125.2
(22) Anmeldetag: 26.02.2009
(51) Int. Cl.: A61M 5/315

(54) **LEERSCHIEßGESCHWINDIGKEITSBEGRENZUNGSBREMSE**
BLANK INJECTION SPEED LIMITING BRAKE
DISPOSITIF DE FREINAGE LIMITANT LA VITESSE D'INJECTION À VIDE

(30) Priorität: 29.02.2008 DE 102008011881
(43) Veröffentlichungstag der Anmeldung: 22.12.2010
(73) Patentinhaber: Tecpharma Licensing AG, 3401 Burgdorf (CH)
(72) Erfinder: EICH, Adrian, CH-3374 Wangenried (CH); HORISBERGER, Aurèle, CH-4123 Allschwil (CH); HOSTETTLER, Patrick, CH-3415 Hasle b. Burgsdorf (CH); KLADIWA, Malte, CH-3008 Bern (CH); MEIER, Stefan, CH-3270 Aarberg (CH); SETTLER, Peter, CH-3422 Kirchberg (CH); WITTMANN, Juergen, CH-3400 Burgdorf (CH)
(86) Internationale Anmeldenummer: PCT/CH2009/000077
(87) Internationale Veröffentlichungsnummer: WO 2009/105908

(56) Entgegenhaltungen:
- EP-A- 0 525 525
- WO-A-03/011370
- WO-A-2008/053243
- WO-A1-00/24441
- WO-A1-03/092771
- WO-A2-2008/112472
- DE-A1- 10 015 616
- DE-C- 426 587
- US-A- 5 320 609

## Beschreibung

Die Erfindung betrifft eine Bremsvorrichtung zum Abbremsen eines sich bewegenden Teils in einer Injektionsvorrichtung zur Verabreichung eines Produkts. Das Produkt ist vorzugsweise ein Medikament, wie z. B. ein Wachstumshormon, Insulin oder dergleichen. Eine solche Injektionsvorrichtung ist bevorzugt mit automatischer Produktausschüttung, wobei ein Energiespeicher, wie z. B. eine Antriebsfeder vorgesehen ist, welche die notwendige Ausschüttenergie beliefert.

Bei Injektionsvorrichtung aus dem Stand der Technik, bei denen ein auf den Kolben eines Produktbehältnisses wirkendes Abtriebsglied mittels einer Feder angetrieben wird, wird in die Funktionsweise der Vorrichtung ein eingesetztes Produktbehältnis mit einkalkuliert. Da das Produkt beim Ausschütten durch einen engen Kanal, wie z. B. eine Nadel, durchfließen muss, wird aufgrund der Viskosität des Produkts eine Dämpfung erzeugt, welche der Kraft des Abtriebsglieds entgegenwirkt und das Abtriebsglied somit auf einen relativ niedrigem Geschwindigkeitsniveau hält. Aufgrund der relativ niedrigen Geschwindigkeit fallen auch die Impulse, wenn ein sich bewegendes Teil an anderes Teil in der Injektionsvorrichtung anschlägt, relativ niedrig aus, was sich positiv auf die Lebensdauer der Vorrichtung auswirkt. Dies setzt jedoch immer voraus, dass ein Produktbehältnis in die Vorrichtung eingesetzt ist. Sobald ein Verwender die Vorrichtung "austricksen" möchte und beispielsweise kein Produktbehältnis einlegt und eine Produktausschüttung startet, fehlt die Dämpfung. Hierbei kann es zu extremen Beschleunigungswerten in der Mechanik der Injektionsvorrichtung kommen, so dass die Impulse von aneinander anschlagenden Teilen mitunter sehr hoch ausfallen kann, was sich negativ auf die Lebensdauer der Injektionsvorrichtung auswirkt. Die Injektionsvorrichtung kann mitunter so stark beschädigt werden, dass sie für weitere Injektionen unbrauchbar sein kann. Eine entsprechend stärkere Dimensionierung erhöht die Baugröße der Vorrichtung, so dass sie klobig erscheinea kann.

Es kann ferner der Fall auftreten, dass nach dem Einsetzen einer neuen Ampulle das Abtriebsglied nicht an dem Kolben der Ampulle anliegt. Dies ist insbesondere der Fall, wenn das Abtriebsglied beim Ampullenwechsel zu weit zurückgeschoben wurde oder wenn z. B. nur eine halb gefüllte Ampulle eingesetzt wird. Auch hier kann es bei dem Leerhub des Abtriebsglieds, insbesondere bei dem Hub, den das Abtriebsglied bis zum Anschlagen auf den Kolben der Ampulle ausführt, wieder zu den extremen Beschleunigungswerten kommen. Ist in solch einem Fall keine Nadel an der Ampulle angeordnet, so wird das Produkt der Ampulle durch den Aufprall des Abtriebsglieds komprimiert, wodurch eine Wechselwirkung mit dem Produkt in der Ampulle, d.h. eine Druckerhöhung in dem Produkt stattfindet, was zu einer ungesteuerten Ausschüttung eines Medikamentes führt wenn die Nadel auf die Injektionsvorrichtung ausgesetzt wird.

Aus dem Stand der Technik sind Bremsmechanismen für Injektionsvorrichchtungen bekannt. WO03/092771 A1 offenbart Bremsvorrichtungen für Autoinjektoren, bei welchen in einer ersten Phase eine Bewegung in der Bremsvorrichtung verhindert wird und in einer zweiten Phase, bei ansteigenden Kräften, eine Bewegung ermöglicht wird. Die Funktion bezieht sich dabei auf Injektionsgeräte mit einem Nadelrückzug, wobei der Nadelrückzug erfindungsgemäss reproduzierbar und zuverlässig erst nach vollständiger Medikamentenausschüttung auslöst. In einer hier beispielhaft angeführten beschriebenen Ausführungsform einer Bremsvorrichtung aus der WO03/092771 A1 wird bei einer Medikamentenausschüttung die Ausschüttkraft von einer Antriebsscheibe auf eine Abtriebsscheibe übertragen, wobei die Kraft durch Haftreibung übertragen wird. Überwindet die resultierende Kraft in der Bremsvorrichtung die Haftreibung, so beginnen sich Antriebsscheibe und Antriebsscheibe relativ zueinander zu bewegen. In der beschriebenen Ausführungsform überwindet die resultierende Kraft in der Bremsvorrichtung dann die Haftreibung, wenn die in der Injektionsvorrichtung vorhandene Spritze vollständig geleert, also der Spritzenkolben am vorderen Ende der Spritze ansteht und sich nicht mehr weiter bewegen kann. Durch die folgendeRelativbewegung zwischen Antriebsscheibe und Abtriebsscheibe kann der Nadelrückzug ausgelöst werden. Die in der WO03/092771 A1 beschriebenen Ausführungsformen können jedoch während eines Ausschüttvorganges extreme Beschleunigungen im Falle von zum Beispiel fehlendem Produktverhältnis nicht verhindem, da die offenbarten Bremsmechanismen erst nach erfolgter Ausschüttung Wirkung entfalten.

Es ist eine Aufgabe der Erfindung, Maßnahmen vorzuschlagen, welche eine Schädigung der Injektionsvorrichtung bei einem nicht eingelegten Produktbehältnis verhindern.

Die Aufgabe wird gelöst durch die Merkmale des Anspruchs 1. Vorteilhafte Weiterentwicklungen ergeben sich aus den abhängigen Ansprüchen.

Die Erfindung betrifft eine Bremsvorrichtung, die insbesondere in einer Injektionsvorrichtung eingebaut sein kann. Die Bremsvorrichtung wirkt auf ein Teil der Injektionsvorrichtung, welches sich bewegt. Die Bewegung kann eine lineare Bewegung sein. Vorzugsweise ist die Bewegung eine Drehbewegung. Bevorzugt wirkt die Bremse auf dem Prinzip, dass eine mechanische Bewegung dissipiert wird, so dass die mechanische Bewegung in eine andere physikalische Größe umgewandelt wird, wie z. B. Wärme. Bevorzugt dämpft der Bremsmechanismus die mechanische Bewegung. Die Dämpfung ist bekanntermaßen abhängig von der Geschwindigkeit. Das heißt, dass bei einer großen Geschwindigkeit die Dämpfung groß ist und bei einer kleinen Geschwindigkeit die Dämpfung entsprechend klein ist. Bevorzugt wird beim erfindungsgemäßen Bremsmechanismus die maximale. Drehgeschwindigkeit oder Winkeigeschwindigkeit eines sich z. B. während eines Ausschilttvorgangs schnell drehenden Teils begrenzt, wie z. B. einer Anzeigetrommel oder einem Übertragungsglied, welches die Bewegung eines Energiespeichers, wie z. B. der Ausschüttfeder, auf das Abtriebsglied überträgt, das für die Ausschüttung auf einem Kolben eines Produktbehältnisses wirken kann. Bei einem eingesetzten Produktbehältnis bewegen sich die Teile des Übertragungselements aufgrund der Dämpfung der in dem Produktbehältnis enthaltenen Flüssigkeit relativ langsam. Jedoch wird durch den Bremsmechanismus maximal eine zu vernachlässigende Dämpfung oder Bremswirkung erzeugt. Bei nicht eingelegtem Produktbehältnis fehlt die Dämpfung des Fluids des Produktbehältnisses, so dass die durch die Ausschüttfeder angetriebenen Teile des Übertragungselements auf z.B. eine höhere Geschwindigkeit beschleunigen als bei einem eingesetzten Produktbehältnis, was dazu führt, dass die Dämpfung oder Bremswirkung des Bremsmechanismus auf einen nicht mehr zu vernachlässigbaren Wert ansteigt.

Bevorzugt ist der Bremsmechanismus so ausgestaltet, dass er als Modul, welches bei einer Entwicklung einer Injektionsvorrichtung auf einfache Weise in die Injektionsvorrichtung a-daptierbar ist, ausgebildet ist.

Eine Bremsvorrichtung oder ein Bremsmechanismus für eine Injektionsvorrichtung zur Erzeugung einer Bremswirkung auf ein sich drehendes Teil der Injektionsvorrichtung kann erfindungsgemäß eine Bremskraft erzeugen, welche so groß ist, dass eine normale Ausschüttbewegung des sich drehenden oder bewegenden Teiles nicht blockiert oder stark behindert wird, wobei die Bremskraft jedoch eine zu schnelle Drehung oder zu starke Beschleunigung oder Bewegung des Teiles verhindert oder abbremst. Insbesondere erzeugt die erfindungsgemäße Bremsvorrichtung eine Bremswirkung auf ein sich z.B. zur Bewirkung einer Dosisabgabe oder Ausschüttung bewegendes oder drehendes Teil und ist unmittelbar verbunden oder alternativ auch gekoppelt mit dem die Ausschüttung bewirkenden Teil, wie z.B. einer Gewindestange oder einer Kolbenstange, so dass eine zu schnelle Drehung der Gewindestange oder zu schnelle Bewegung der Kolbenstange, welche z.B. beim so genannten Leerschießen auftritt, verhindert werden kann.

Es ist auch möglich, dass die Bremsvorrichtung nicht unmittelbar auf das die Abgabe bewirkende Teil, wie z.B. eine Gewindestange oder Kolbenstange, sondern auf ein damit gekoppeltes Teil, wie z.B. ein Teil eines Übertragungselements oder eines Antriebsstrangs, eine Anzeigetrommel, eine Gewindehülse, eine Antriebswelle oder eine Bremsbacke einwirkt.

Gemäß einer ersten Ausführungsform wird die Bremsvorrichtung durch ein erstes Bremselement, wie z.B. eine Bremsscheibe gebildet, welche durch das Zusammenwirken mit mindestens einem zweiten Bremselement, wie z.B. einem an der Bremsscheibe anliegenden Element bei Bewegung oder Drehung des Elements oder der Bremsscheibe eine Bremskraft erzeugen kann. Dabei kann das Bremselement z.B. an einem Gegenelement reiben, um als eine an sich bekannte durch Reibungskraft wirkende Bremse für die gewünschte Abbremsung zu sorgen. Dabei wird Dreh- oder Bewegungsenergie in Wärme umgesetzt.

Alternativ ist es auch möglich, dass das erste Bremselement ein Profil aufweist, wie z.B. ein stirnseitig an einer oder zwei gegenüberliegenden Seiten der Bremsscheibe vorgesehenes Zahn-Profil. Diese Verzahnung der Bremsscheibe kann in eine oder zwei z.B. einander gegenüberliegende bevorzugt fest beabstandete Gegenverzahnungen oder Gegenprofile eingreifen, wobei ein Spiel zwischen der Gegenverzahnung und einer Anlage oder zwischen zwei Gegenverzahnungen vorgesehen ist, so dass die Bremsscheibe zwischen der Gegenverzahnung und der Anlage zwischen den beiden Gegenverzahnungen oszillieren, also sich z.B. hin- und herbewegen kann, wenn eine relative Drehbewegung zwischen der Bremsscheibe und der Gegenverzahnung bzw. den Gegenverzahnungen vorliegt. Dabei kann sowohl die Bremsscheibe als drehendes Teil ausgebildet sein, als auch eine oder beide der Gegenverzahnungen, welche z.B. mit einer Gewindestange gekoppelt oder verbunden sein können. Vorzugsweise ist die Bremsscheibe oder sind die Gegenelemente verdrehsicher oder verschiebbar in z.B. die Richtung der Mittelachse der Bremsscheibe gelagert.

Gemäß einer weiteren alternativen Ausführungsform kann die Bremse auch als Fliehkraftbremse realisiert sein, wobei an einem drehenden Teil oder mit dem drehenden Teil gekoppelt ein oder mehrere Masseelemente vorgesehen sein können, welche z.B. radial nach innen z.B. durch die Kraft einer Feder vorgespannt sein können. Wird das sich bei einem Ausschüttvorgang drehende Teil in Bewegung gesetzt, so bewegen sich die Massen entgegen der radial nach innen wirkenden Federkraft nach außen und können z.B. mit einem Gegenelement, wie z.B. einer Hülse, an mindestens einem und vorzugsweise mehreren Bereichen während ihres Umlaufs in Anlage kommen und können z.B. auch durchgängig in der durch die Zentrifugalkraft ausgeschobenen Stellung an dem Gegenelement anliegen, um so z.B. durch Reibung für die gewünschte Abbremsung zu sorgen. Dabei kann z.B. an der Außenseite der Massen ein Bremselement, wie z.B. ein Gummibelag oder eine andere geeignete Beschichtung, aufgebracht sein, um die Bremswirkung zu erhöhen. Ebenso ist es möglich z.B. Permanentmagneten aufzubringen, mit welchen die Haftung zwischen den Masseelementen und einer z.B. magnetischen oder ferromagnetischen äußeren Umhüllung zur Vergrößerung der Bremswirkung erhöht wird.

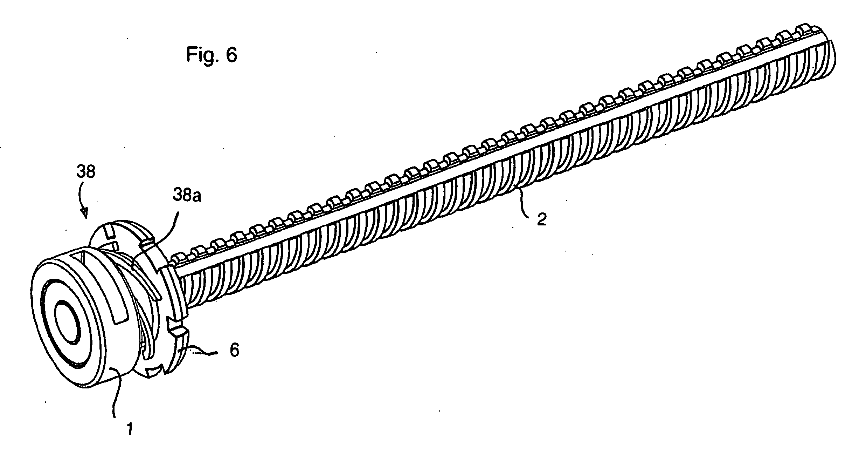

Nach einer weiteren Ausführungsform kann die Bremsvorrichtung als Wirbelstrombremse ausgebildet sein, wie nachfolgend unter Bezugnahme auf Figur 6 beschrieben.

Gemäß einer weiteren Alternative ist es möglich die Bremsvorrichtung als Fluidbremse auszubilden, bei welcher sich z.B. ein mit dem sich drehenden oder bewegenden Teil verbundenes Element in einem mit einem Fluid gefüllten Volumen bewegt, so dass durch die Bewegung des Elementes in dem Fluid die gewünschte Brems- oder Gegenkraft erzeugt wird.

Die Erfindung wurde anhand mehrerer Ausführungsformen beschrieben. Im Folgenden wird eine Ausführung der Erfindung anhand von Figuren beschrieben. Die hierbei offenbarten Merkmale bilden je einzeln und in Kombination den Gegenstand der Erfindung vorteilhaft weiter. Es zeigen:
- Figur 1: eine Querschnittsansicht eines proximalen Teils einer Injektionsvorrichtung,
- Figur 2: eine perspektivische Ansicht eines Gehäuseteils mit einer Führungsbahn für einen Bajonettverschluss und einer eingesetzten Bajonetthülse,
- Figur 3: eine perspektivische Ansicht der Bajonetthülse aus Figur 2
- Figur 4: eine perspektivische Ansicht der Bajonetthülse aus Figur 3 mit einem darin eingesetzten Eingriffsglied
- Figur 5: eine perspektivische Ansicht einer Bajonetthülse und einer Produktbehältnisaufnahme, die axial in einen drehmomentfesten Eingriff gebracht sind,
- Figur 6: eine perspektivische Ansicht eines Abtriebsglieds mit Flansch und Federglied,
- Figur 7A und 7B: eine Explosionsansicht und eine perspektivische Ansicht einer Bremseinrichtung gemäß einer ersten Ausführungsform,
- Figur 8: eine perspektivische Explosionsansicht einer Bremseinrichtung gemäß einer zweiten Ausfiihrungsform,
- Figur 9: ein Diagramm mit einem schematischen Verlauf der Bremskraft in Abhängigkeit von der Winkelgeschwindigkeit,
- Figur 10: ein Diagramm zur Erläuterung der Bremswirkung bei einer zeitabhängigen Darstellung,
- Figuren 11A und 11B: eine Explosionsansicht und eine perspektivische Ansicht einer auf dem Prinzip einer Wirbelstrombremse wirkenden Bremseinrichtung gemäß einer dritten Ausftihrungsform,
- Figur 12: eine perspektivische Ansicht einer auf dem Prinzip einer Fliehkraftbremse wirkenden Bremseinrichtung gemäß einer vierten Ausführungsform,
- Figur 13: eine Explosionsansicht einer auf dem Prinzip einer Fluidbremse wirkenden Bremseinrichtung gemäß einer fünften Ausführungsform und
- Figur 14: eine perspektivische Ansicht eines Bremsengehäuses aus Figur 13.

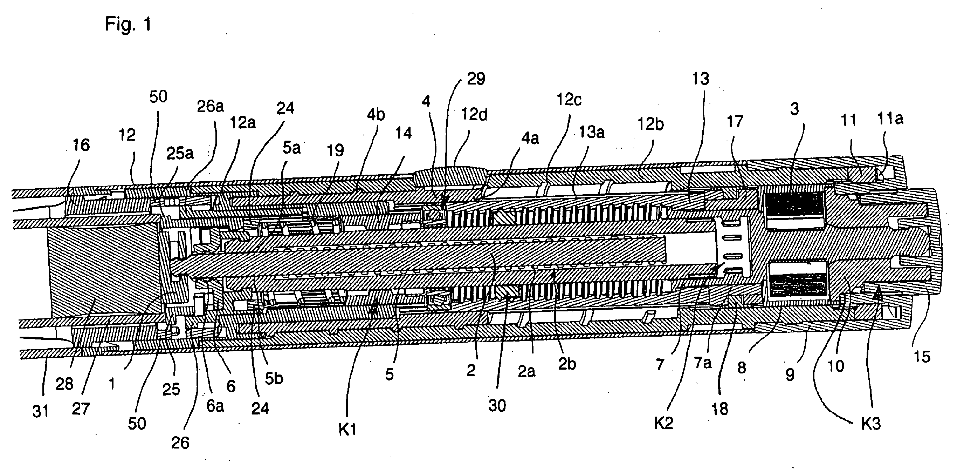

Die in Figur 1 gezeigte Injektionsvorrichtung umfasst eine Antriebseinheit, die bevorzugt mehrfach verwendbar ist, und ein damit verbundenes Produktbehältnis 27, das in einer hülsenförmigen z.B. mehrfach verwendbaren Produktbehältnisaufnahme 16 aufgenommen und mit Hilfe der Produktbehältnisaufnahme 16 an der Antriebseinheit befestigbar ist. Das Produktbehältnis 27 kann nach seiner Entleerung von der Injektionsvorrichtung entfernt, entsorgt und gegen ein neues ausgetauscht werden. Das Gehäuse 12 ist wegen der einfacheren Herstellbarkeit und Montierbarkeit mehrteilig gebildet mit damit verbundenen oder eingesetzten Elementen 12a, 12b, wobei das Gehäuse grundsätzlich auch einteilig gebildet sein könnte. Das Produktbehältnis 16 ist mit Hilfe eines Bajonettverschlusses, der von dem Gehäuse 12, der Produktbehältnisaufnahme 16 und der Hülse 50 gebildet wird, an der Antriebseinheit befestigt. Die Produktbehältnisaufnahme 16 wird durch eine Kappe 31 abgedeckt, die an das Gehäuse 12 angesteckt, für eine Verwendung der Injektionsvorrichtung abnehmbar und anschließend wieder aufsteckbar ist.

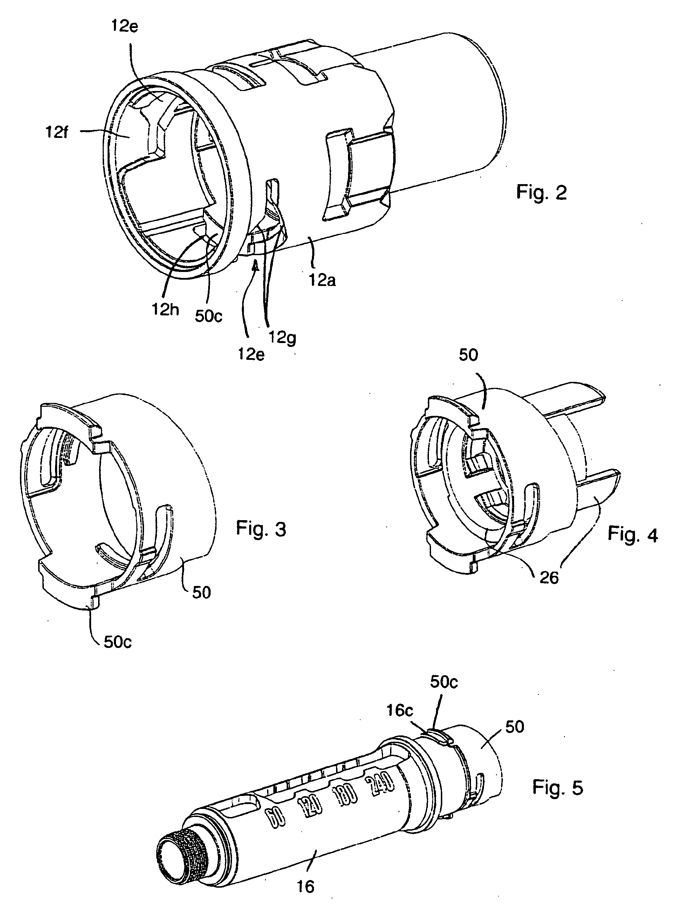

Die Figuren 2 bis 5 zeigen wesentliche Elemente der als Bajonettverschluss ausgestalteten Befestigungseinrichtung. Die Produktbehältnisaufnahme 16 weist einen radial nach außen ragenden Nocken 16c auf und ist an ihrer proximalen Stirnseite so gebildet, dass sie formschlüssig, d.h. drehmomentfest mit der distalen Stirnseite der Hülse 50 verbindbar ist, wie in Figur 5 gezeigt, in der zu Darstellungszwecken das Gehäuseteil 12a weggelassen wurde. Die Hülse 50 weist mindestens einen radial nach außen ragenden Nocken 50c auf, der einen Teil eines Nockens 16c, 50c für die Befestigungseinrichtung bildet. Der Nocken 50c greift in eine in dem Gehäuse 12, insbesondere in dem Gehäuseteil 12a gebildete Führungsbahn 12e, die mindestens eine schräge Fläche 12g aufweist, ein. Bei einer Drehbewegung der Hülse 50 wird die Hülse 50 aufgrund des Eingriffs des Nockens 50c zusätzlich zu der Drehbewegung relativ zu dem Gehäuseteil 12a axial bewegt. Durch die Axialbewegung der Hülse 50 können, wie noch beschrieben wird, verschiedene vorteilhafte Wirkungen erzielt werden.

Zum Anbringen des Produktbehältnisses 27 an die Antriebseinheit wird ein neues Produktbehältnis 27 durch das proximale Ende in die Produktbehältnisaufnahme 16 eingeführt. Anschließend wird die Produktbehältnisaufnahme 16 mit einer axialen Bewegung drehmomentfest an die Hülse 50 angesteckt (Figur 5), wobei die Nocken 16c durch die Öffnung 12f (Figur 2) in die Führungsbahn 12e eingeführt werden. Figur 2 zeigt den Bajonettverschluss ohne die Produktbehältnisaufnahme 16 in einem verriegelten Zustand. In einem entriegelten Zustand, in dem sich die Nocken 50c im Bereich, insbesondere in einer axialen Flucht der Öffnungen 12f befinden, kann die Produktbehältnisaufnahme 16 angesteckt werden. Die Nocken 16c und 50c liegen dann aneinander an und bilden quasi einen gemeinsamen Nocken 16c, 50c (Figur 5). Eine Verdrehung der Produktbehältnishalterung 16 bewirkt eine Mitnahme des Hülse 50. Durch die Schrägen 12g werden die Hülse 50 und die Produktbehältnisaufnahme 16 zusätzlich axial bewegt. Am Ende der Drehung, d.h. beim Erreichen der verriegelten Position befindet sich der gemeinsame Nocken 16c, 50c im Bereich 12h der Führungsbahn 12e, in dem die beiden Nocken 16c und 50c von den Flanken der Führungsbahn 12e axial gegeneinander gespannt werden. Hierzu ist die axiale Breite der Führungsbahn im Bereich 12h in etwa so breit, wie die des gemeinsamen Nockens 16c, 50c.

Wie in Figur 4 gezeigt wird, ist in der Hülse 50, die auch als Bajonetthülse bezeichnet werden kann, eine Führungshülse 26 aufgenommen. Die Führungshülse 26 ist mit dem Gehäuse 12 drehfest und axial bewegbar sowie mit der der Bajonetthülse 50 drehbar und axial fest verbunden. Dies bewirkt, dass bei der Bewegung der Bajonetthülse 50 aus der entriegelten in die verriegelte Position und umgekehrt, die Führungshülse 26 eine längsgeführte Bewegung relativ zum Gehäuse 12 ausfuhrt.

Wie in Figur 1 zu erkennen ist, ist ein Gewindeeinsatz 6 dreh- und axialfest mit der Führungshülse 26 verbunden, insbesondere verrastet. Gewindeeinsatz 6 und Führungshülse 26 können als Eingriffsglied 6, 26 bezeichnet werden. Der Gewindeinsatz 6 weist ein Innengewinde 6a auf, in welchem das Außengewinde 2a eines Abtriebsglieds 2, das in diesem Beispiel auch als Kolbenstange bezeichnet werden kann, geführt wird, so dass, wenn das Abtriebsglied 2 gedreht wird, sich dieses geführt durch das Innengewinde 6a des Gewindeeinsatzes 6 in Abhängigkeit von der Drehrichtung entweder in proximale Richtung oder in distale, d.h. entgegengesetzte Richtung schraubt.

Das Abtriebsglied 2 trägt auf seiner Außenseite ein Gewinde 2a welches von zwei am Umfang gegenüberliegenden, in axiale Richtung verlaufenden Nuten 2b unterbrochen wird. Eine Kupplungshülse 5, die Teil eines Übertragungselements 7, K2, 5 ist, hat an ihrem distalen Ende zwei einander gegenüberliegende radial nach innen gerichtete Abragungen 5a, 5b, welche in die Nuten 2b des Abtriebsglieds 2 ragen. Die Kupplungshülse 5 ist mit dem Eingriffsglied 6, 26 drehbar und axial fest verbunden. Somit ist das Abtriebsglied 2 relativ zu der Kupplungshülse 5 verdrehgesichert und kann relativ zur Kupplungshülse 5 axial bewegt werden, wenn es relativ zu dem Eingriffsglied 2, 26 gedreht wird. Außer während eines Austauschs des Produktbehältnisses 27 ist die Kupplungshülse 5 axial nicht verschiebbar.

Eine an dem proximalen Ende der Injektionsvorrichtung vorgesehene Antriebswelle 7, die Teil des Übertragungselements 7, K2, 5 ist, weist radial nach innen ragende Zähne 7a auf, welche ein Kupplungselement der Kupplung K2 bilden. Durch das Betätigungen, d.h. Drücken eines Betätigungselements 15 in distale Richtung, werden die Antriebswelle 7 und dadurch auch die Zähne 7a in distale Richtung verschoben, wodurch die Zähne 7a in das proximale Ende der Kupplungshülse 5 eingreifen und eine drehmomentfeste, insbesondere formschlüssige Verbindung bilden.

Ein Federelement bzw. eine Antriebsfeder 3, welche in Form einer Spiralfeder oder Uhren-Feder ausgebildet sein kann, ist mit einem Ende mit dem Gehäuse 12 über einer Federhülse 8 auf der Außenseite des Federelements 3 verbunden. Die Federhülse 8 ist relativ zu dem Gehäuse 12 verdrehgesichert und axial verschiebbar. Am anderen Ende ist die Antriebsfeder 3 mit der Antriebswelle 7 verbunden. Hierdurch kann eine in dem Federelement 3 gespeicherte Energie als Drehbewegung der Antriebswelle 7 relativ zu dem Gehäuse 12 abgegeben werden. Für eine Produktausschüttung wird die Energie des Federelements 3 über das Übertragungselement 5, K2, 7 in Form einer Drehbewegung an das Abtriebsglied abgegeben, so dass sich dieses relativ zu dem Eingriffsglied 6, 26 in distale Richtung, d.h. in Ausschüttrichtung schraubt und den Kolben 28 verschiebt, der das Produkt aus dem Produktbehältnis 27 ausschüttet.

Zum Einstellen einer zu verabreichenden Produktdosis kann ein Benutzer das als Dosierknopf ausgestaltete Dosierelement 9, das relativ zu dem Gehäuse 12 axialfest ist, drehen. Das Dosierelement 9 ist über die Kupplung K3 mit einem Kupplungsglied 10 verdrehgesichert gekoppelt. Die Kupplung K3 wird gebildet durch Stege oder Nuten oder Zähne des Dosierknopfes 9, welche mit Stegen oder Nuten oder Zähnen der Kupplungsscheibe 10 formschlüssig zusammenwirken, um eine durch Verschieben des Kupplungsglieds 10 in distale Richtung lösbare Kupplung zu bilden. Das Kupplungsglied 10 kann durch Betätigen des Betätigungselements 15 verschoben und somit gelöst werden. In einem unbetätigten Zustand werden die Kupplungen K3 in einem eingekuppelten und die Kupplung K2 in einem ausgekuppelten Zustand gehalten mittels eines Federelements 19, welches die Antriebswelle 7 in proximale Richtung drückt. Während des Dosiseinstellvorgangs ist die Kupplung K3 eingekuppelt, d.h. eine Drehbewegung des Dosierknopfes 9 wird auf das Kupplungsglied 10 übertragen. Das Kupplungsglied 10 ist mit der Antriebswelle 7 axial- und drehfest verbunden und könnte auch einteilig mit der Antriebswelle 7 ausgebildet sein. Die Drehbewegung des Dosierglieds 9 wird aufgrund der ausgekuppelten Kupplung K2 nicht auf die Kupplungshülse 5 übertragen.

Durch Drehung der Antriebswelle 7 wird die mit der Antriebswelle 7 verbundene Antriebsfeder 3 gespannt. Um zu verhindern, dass durch die während des Einstellvorganges gespannte Antriebsfeder 3 der Dosierknopf 9 wieder zurückgedreht wird, ist eine Ratsche 11 oder ein Ratschmechanismus, welcher eine Ratschenfeder 11a z.B. zum Spannen von Halteelementen aufweisen kann, zwischen dem Gehäuse 12 der Injektionsvorrichtung, dessen Bestandteile z.B. ein Mechanikhalter 12a und ein Mechanikhalter 12b sein können, und dem Dosierknopf 9 vorgesehen. Der Ratschenmechanismus kann so ausgestaltet sein, dass nur die Drehung in eine Richtung, insbesondere nur ein Spannen der Antriebsfeder 3 möglich ist. Bevorzugt wird jedoch, dass der Ratschenmechanismus so ausgestaltet ist, dass die Drehung in beide Drehrichtung, insbesondere ein Spannen und Entspannen der Antriebsfeder 3 möglich ist. Durch eine Drehbarkeit in beide Richtungen kann bei der Einstellung der Produktdosis sowohl eine Produktdosis erhöht als auch verringert werden. Die aktuell eingestellte Produktdosis kann über das Fenster 12d von einer Anzeigetrommel 4 abgelesen werden.

Die Drehbewegung der Antriebswelle 7 wird auch auf die Gewindehülse 13 übertragen, welche dreh- und axialfest mit der Antriebswelle 7 verbunden ist und auch einteilig mit dieser ausgebildet sein könnte. Die Gewindehülse 13 trägt auf ihrem äußeren Umfang 13a mindestens eine Nut, in welche mindestens ein Steg 4a der Anzeigetrommel 4 eingreift, so dass eine Drehbewegung der Gewindehülse 13 auf die Anzeigetrommel 4 durch die verdrehsichere Kopplung übertragen wird, wobei eine axiale Relativbewegung zwischen Anzeigetrommel 4 und Gewindehülse 13 möglich ist. Die Anzeigetrommel 4 weist auf ihrer Außenseite ein Gewinde 4b auf, welches in ein Innengewinde 12c des Gehäuseteils 12b eingreift, so dass die Anzeigtrommel 4 durch eine Drehbewegung in axiale Richtung relativ zum Gehäuse 12 vorzugsweise in distale Richtung verschoben wird. Bevorzugt bewegt sich die Anzeigetrommel 4 während eines Einstell- oder Aufdosiervorganges durch Drehung des Dosierknopfes 9 in distale Richtung der Injektionsvorrichtung (in Fig. 1 nach links). Auf der Außenseite der Anzeigetrommel 4 kann eine Markierung, wie z.B. eine Beschriftung, eine Dosisanzeige oder eine Skala vorgesehen sein, welche durch eine Durchbrechung oder ein Fenster 12d im Gehäuse 12b der Injektionsvorrichtung ablesbar ist, wobei sich die Markierung der Anzeigetrommel 4 relativ zum Fenster 12d verschiebt. Die Anzeigetrommel 4 weist an seinem distalen Ende einen in Umfangsrichtung wirkenden Drehanschlag auf, der bei maximaler Dosis in einen Anschlag mit einem entsprechend an dem Gehäuseteil 12a ausgebildeten Gegenanschlag gerät. Der Gegenanschlag wird von einem stirnseitigen Ende eines Ringspalts des Gehäuseteils 12a gebildet. Ein in Umfangsrichtung wirkender Anschlag hat gegenüber einem Axialanschlag den Vorteil, dass geringere Kräfte auf den Anschlag wirken. Die Anzeigetrommel 4 weist ferner an seinem proximalen Ende einen weiteren in Umfangsrichtung wirkenden Drehanschlag auf, der bei minimaler Dosis in einen Anschlag mit einem entsprechend an dem Gehäuse 12b ausgebildeten Gegenanschlag gerät. Der Gegenanschlag wird von dem proximalen Ende des Gewindegangs 12c gebildet.

Nach dem Einstellen der Dosis und Aufziehen der Antriebsfeder 3 durch Drehen des Dosierknopfes 9 ist der Einstellvorgang beendet, wobei bevorzugt ist, dass das Spannen der Feder 3 beim Aufdosieren erfolgt. Zur Dosiskorrektur kann der Dosierknopf 9 einfach in Gegenrichtung gedreht werden, um eine eventuell zu groß eingestellte Dosis wieder zu verkleinern.

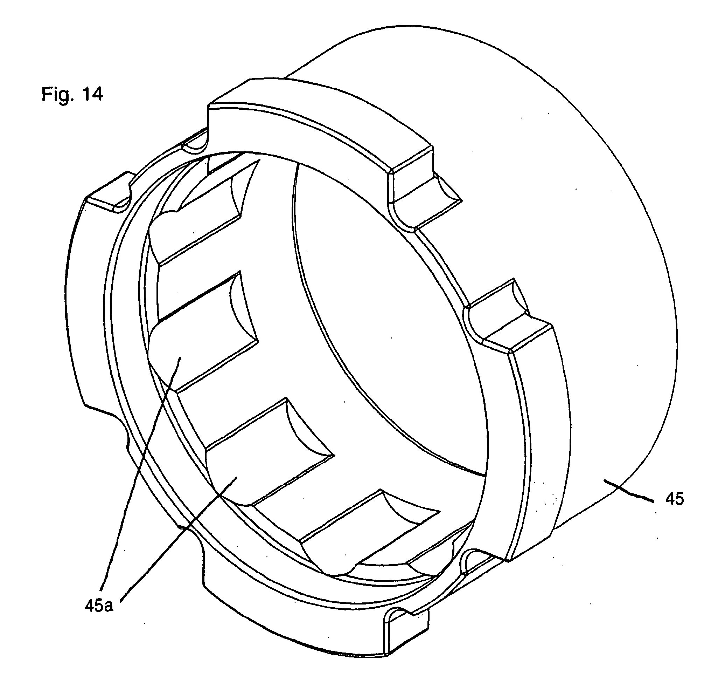

Die Ratsche 11 kann so ausgebildet sein wie in den Figuren 14 und 15 der Patentanmeldung PCT/CH2007/000243 beschrieben, deren diesbezügliche Lehre in die Patentanmeldung aufgenommen wird.

Während eines Ausschüttvorganges, welcher durch Drücken auf den Druckknopf 15 ausgelöst wird, wird die Anzeigetrommel 4 wieder in Gegenrichtung gedreht und verschiebt sich durch den Gewindeeingriff mit dem Innengewinde 12c der Injektionsvorrichtung wieder zurück in proximale Richtung (in Fig. 1 nach rechts). Dabei kann es auch zu einem in Umfangsrichtung wirkenden Anschlag der Anzeigetrommel 4 an das Gehäuse 12a, 12b der Injektionsvorrichtung und insbesondere an dem Gehäuseteil 12b kommen. Dieser Vorgang kann bei einer ungebremsten Ausschüttbewegung, bei welcher die Gewindestange 2 ohne Gegenkraft in distale Richtung bewegt wird, z.B. wenn kein Produktbehältnis eingelegt ist, zu einer starken Belastung und im Extremfall zu einer Verformung oder sogar Beschädigung der Anzeigetrommel 4 oder des Gegenstücks 12b führen. Es ist daher eine auf die Antriebsbewegung wirkende Bremseinrichtung 17, 18 vorgesehen, die weiter unten beschrieben wird.

Die Kupplung K1, die aus dem als Arretierhülse 14 ausgestalteten Kupplungsglied und der Kupplungshülse 5 gebildet wird, dient dazu, in bestimmten Schaltzuständen die Kupplungshülse 5 drehfest mit dem Gehäuse 12 zu koppeln bzw. für eine Drehung relativ zu dem Gehäuse 12 zu entkoppeln. Die Kupplung K1 ist bevorzugt entkoppelt bei einem Austausch des Produktbehältnisses 27, um das Abtriebsglied 2 wieder in proximale Richtung zurückschieben bzw. schrauben zu können, und bei einer Produktausschüttung, um das Abtriebsglied 2 in distale Richtung schrauben zu können. Die Kupplung K1 ist bevorzugt eingekuppelt, wenn das Produktbehältnis an der Antriebseinheit befestigt ist und das Betätigungselement 15 unbetätigt ist. Die Kupplung K1 wird gebildet durch Zähne auf der Außenseite der Kupplungshülse 5, welche in Zähne auf der Innenseite der Arretierhülse 14 eingreifen. Hierdurch wird die Kupplungshülse 5 relativ zur Arretierhülse 14 verdrehgesichert. Die Arretierhülse 14 ist verdrehgesichert und axial verschiebbar in der Injektionsvorrichtung, insbesondere relativ zu dem Gehäuse 12 und der Kupplungshülse 5 gelagert.

Während eines Ausschüttvorganges wird die Gewindehülse 13 durch Betätigung des Betätigungselements 15 in distale Richtung verschoben. Dabei drückt die Gewindehülse 13 auf das Lager 29, das in diesem Beispiel als Kugellager ausgebildet ist, jedoch auch als einfaches Gleitlager ausgebildet sein kann, wobei das Lager 29 auf die Arretierhülse 14 drückt, diese somit für einen Ausschüttvorgang in distale Richtung verschiebt und während eines Ausschüttvorgangs in distaler Position hält. Das Kupplungsglied 14 befindet sich somit distal der Abragungen der Kupplungshülse 5 für die Kupplung K1. Dadurch ist die Kupplung K1 für die Dauer des Ausschüttvorganges ausgekuppelt.

Beim Betätigen des Betätigungselements 15 verhalten sich die Kupplungen K1, K2 und K3 folgendermaßen: Durch Drücken des auf dem Kupplungsglied 10 und/oder Antriebswelle 7 sitzenden Druckknopfs 15 werden das Kupplungsglied 10 zusammen mit dem Druckknopf 15 und die Antriebswelle 7 in distale Richtung verschoben. Hierdurch kuppelt die Kupplung K2 ein, so dass die Antriebswelle 7 mit der Kupplungshülse 5 verdrehgesichert wird. Anschließend kuppelt die Kupplung K1 durch Verschiebung der Arretierhülse 14, auf welche die mit der Antriebswelle 7 verbundene Gewindehülse 13 über das axial verschiebbare Lager 29 drückt, aus. Die Kupplungen K1 und K2 können alternativ auch in umgekehrter Reihenfolge geschaltet werden.

Nach dem Einkuppeln von K2 und dem Auskuppeln von K1 kuppelt auch die Kupplung K3 durch Verschieben des Kupplungsglieds 10 relativ zum Dosierknopf 9 aus. Das Kupplungsglied 10, welches mit der Antriebswelle 7 verbunden ist, kann sich nach dem Auskuppeln der Kupplung K3 relativ zu dem Gehäuse 12 drehen. Die in der Antriebsfeder 3 während des Aufdosierens gespeicherte Energie oder Kraft kann auf die Antriebswelle 7 übertragen werden. Somit liegt an der Antriebswelle 7 ein Drehmoment an, welches mittels der eingekuppelten Kupplung K2 auf die Kupplungshülse 5 übertragen wird, welche sich zusammen mit der. Antriebswelle 7 dreht und diese Drehbewegung auf das verdrehsicher mit der Kupplungshülse 5 gekoppelte Abtriebsglied 2 überträgt. Das in diesem Beispiel als Gewindestange ausgestaltete Abtriebsglied 2 setzt die Drehbewegung aufgrund des Gewindeeingriffs 2a, 6a mit dem Eingriffsglied 6, 26 in eine axiale Bewegung in distale Richtung um, so dass der am distalen Ende der Gewindestange 2. vorgesehene Flansch 1, der ebenfalls zum Abtriebsglied zugerechnet werden kann, in distale Richtung der Injektionsvorrichtung bewegt wird.

Da sich bei der Produktausschüttung die Gewindehülse 13 in entgegen gesetzte Richtung wie beim Aufdosieren dreht, dreht sich die Anzeigetrommel 4 ebenfalls in entgegen gesetzte Richtung wie beim Aufdosieren.

Im Normalfall, d.h. in dem Fall, bei dem die voreingestellte Produktdosis vollständig ausgeschüttet wird, verläuft der Ausschüttvorgang und insbesondere die Verschiebung des Abtriebsglieds 2 in distale Richtung so lange, bis der oben erwähnte in Umfangsrichtung wirkende Anschlag der Anzeigetrommel 4 anschlägt. Dies geschieht bevorzugt dann, wenn sich der durch das Fenster 12d ablesbare Wert auf 0 heruntergedreht hat.

In dem Fall, in dem der Benutzer der Vorrichtung das Beätigungselement 15 während der Produktausschüttung loslässt, kuppeln die Kupplungen in umgekehrter Reihenfolge, wie sie bei der Betätigung aus- bzw. eingekuppelt haben. Die Produktausschüttung wird unterbrochen, wobei durch das Fenster 12d der Wert ablesbar ist, der noch auszuschütten wäre, dass die voreingestellte Dosis komplett ausgeschüttet sein würde. Die Produktausschüttung kann dadurch fortgesetzt werden, dass das Betätigungselement 15 erneut gedrückt wird, wobei die Ausschüttung durch Loslassen des Betätigungselements 15 wieder gestoppt werden kann oder gewartet werden kann, bis die Produktausschüttung vollständig erfolgt ist.

Für den Fall, dass sich im Produktbehältnis weniger Produkt befindet als maximale Dosis auf der Anzeigetrommel angegeben ist, weist die in diesem Beispiel gezeigte Injektionsvorrichtung eine zusätzliche Einrichtung zur Begrenzung der letztmalig einstellbaren maximalen Dosis auf, um zu verhindern, dass eine größere Produktdosis eingestellt werden kann als sich Produkt in dem Produktbehältnis befindet. Hierzu ist ein Läufer 30 vorgesehen, der die Kupplungshülse 5 zumindest teilweise umgreift und in solch einem Eingriff mit der Kupplungshülse 5 ist, dass der Läufer 30 relativ zu der Kupplungshülse 5 drehfest und axial verschiebbar ist. Der Läufer 30 greift ferner mit einem an seinem äußeren Umfang gebildeten Gewinde in ein Innengewinde der Gewindehülse 13 ein. Diese Anordnung bewirkt, dass bei einer relativen Drehung zwischen Gewindehülse 13 und Kupplungshülse 5 eine Axialbewegung des Läufers 30 stattfindet, wobei bei keiner relativen Drehung der Läufer 30 keine Axialbewegung ausführt. Beim Einstellen einer Produktdosis wird die Gewindehülse 13 relativ zu der Kupplungshülse 5 verdreht, so dass der Läufer 30 in proximale Richtung wandert. Beim Ausschütten hingegen, findet keine Relativbewegung zwischen Kupplungshülse 5 und Gewindehülse 13 aufgrund des Kupplungseingriffs der Kupplung K2 statt. Die Läufer führt dann keine Bewegung aus. Nach mehrmaligem Dosieren und Produktausschütten gerät der Läufer 30 in einen axialen Anschlag mit der Antriebswelle 7, wodurch eine weitere Dosiserhöhung nicht mehr möglich ist, auch dann nicht mehr, wenn die Anzeige 4, 12d eigentlich mehr zuließe.

Der Verwender kann das Produktbehältnis 27 gegen ein neues austauschen. Hierzu entfernt er den Produktbehältnishalter 16 durch Drehung relativ zu dem Gehäuse 12 von der Antriebseinheit. Bei der Bewegung aus der befestigten Position in die unbefestigte Position des Produktbehältnisses 27, insbesondere beim Entriegeln des Bajonettverschlusses wird das Eingriffsglied 6, 26 zusammen mit dem Abtriebsglied 2 und der Kupplungshülse 5 relativ zu dem Gehäuse 12 und dem Kupplungsglied 14 in distale Richtung verschoben, wodurch die Kupplung K1 gelöst wird. Die für die Kupplung K1 vorgesehenen radial nach außen weisenden Abragungen der Kupplungshülse 5 befinden sich nun distal des Kupplungsglieds 14. Das Abtriebsglied 2 kann nun mit einer relativ geringen in proximale Richtung wirkenden Kraft in die Antriebseinheit geschraubt werden, da das Gewinde des Abtriebsglieds nicht selbsthemmend ist. Beim Zurückschrauben des Abtriebsglieds 2 wird die Kupplungshülse 5 relativ zu der Gewindehülse 13 und zwar entgegensetzt wie bei der Produktausschüttung verdreht, wodurch der Läufer 30 wieder axial verschoben wird in distale Richtung. Das Zurückschrauben kann zumindest über einen Teil des Gesamtwegs gegen die Kraft eines Federglieds erfolgen, das z.B. versucht, das Abtriebsglied in distale Richtung zu verschieben. Das Federglied kann z.B. zwischen dem Abtriebsglied 2 und der Antriebswelle 7 wirken bzw. angeordnet sein. Weitere vorteilhafte Federglieder werden insbesondere zu Figur 6 weiter unten beschrieben. Allgemein bevorzugt wird, dass die Kraft eines solchen Federglieds geringer ist als die für eine Wechselwirkung über den Kolben auf das Produkt von dem Abtriebsglied 2 erforderliche Kraft

Ferner wird beim Entfernen des Produktbehältnisses 27 das Halteglied 25, das dazu dient, das Produktbehältnis 27 in der Produktbehältnishalterung 16 zu fixieren, von der Feder 19 in distale Richtung verschoben bis es in einen Anschlag mit dem Eingriffsglied 6, 26 gerät. Dieser Anschlag verhindert, dass die Feder 19 sich bei entferntem Produktbehältnis 27 nicht vollständig entspannen kann. Dies ist vorteilhaft, da die Feder 19 auch bei einem entfernten Produktbehältnis 27 genügend Kraft aufbringen sollte, die Kupplung K3 in einem Kupplungseingriff zu halten.

Gemäß einem weiteren Aspekt kann ein gefederter Flansch realisiert werden, wie z.B. in Fig. 6 gezeigt.

Nach einem Wechsel des bevorzugt als Ampulle oder Karpulle ausgestalteten Produktbehältnisses 27 wird der Anwender, wie in der Bedienungsanleitung beschrieben, zum so genannten Entlüften aufgefordert. Dies ist erforderlich, da sich zum einen Luft in dem Produktbehältnis 27 befindet und zum anderen das Abtriebsglied 2 zuvor vollständig in die Antriebseinheit hineingeschoben wurde und durch den unterschiedlichen Füllstand des Produktbehältnisses 27 etwas Spiel zwischen dem Kolben 28 und dem Flansch 1 entstanden ist.

Figur 6 zeigt ein Abtriebsglied 2 mit einer seinem vorderen oder distalen Ende befestigten Flansch 1, der unverschiebbar mit der Gewindestange verbunden ist. Zwischen dem Flansch 1 und dem in Figur 6 gezeigten Gewindeeinsatz 6 ist ein Federglied 38 vorgesehen, welches z.B. durch schräg abstehende Federarme 38a realisiert werden kann. Diese Federarme 38a können am Flansch 1 oder/und am Gewindeeinsatz 6, befestigt sein. Ebenso könnte auch ein Elastomer am Flansch 1 oder/und am Gewindeeinsatz 6 angespritzt werden. Nach dem Einsetzen eines neuen Produktbehältnisses 27 kann es zu einem Spiel zwischen dem Flansch 1 und dem Kolben 28 kommen, was insbesondere auf unterschiedliche Füllstände bei vollen Produktbehältnissen 27 zurückzuführen ist, die eine gewisse Toleranz aufweisen.

Nach dem Einschieben des mit der Gewindestange 2 verbundenen Flansches 1 liegt der Flansch 1 gemäß der in Figur 1 gezeigten Ausführungsform unmittelbar an dem Gewindeeinsatz 6 an.

Gemäß der in Figur 6 gezeigten Ausführungsform wird der Flansch 1 jedoch durch das mindestens eine Federglied 38 vom Gewindeeinsatz 6 um eine vorgegebene Distanz in distale Richtung weggedrückt. Dies ermöglicht es, dass bei einem eingesetzten Produktbehältnis 27 oder beim Einsetzen des Produktbehältnisses 27 der Flansch 1 immer auf der proximalen Seite des Kolbens 28 zum Anliegen kommt, selbst wenn der Kolben 28 bei verschiedenen Produktbehältnissen bedingt durch Fertigungstoleranzen unterschiedlich weit in das Produktbehältnis 27 eingeschoben ist.

Herkömmliche Maßnahmen zur Beseitigung des Spiels zwischen Flansch 1 und Kolben 28 sind daher nicht mehr zwingend erforderlich und können z.B. sogar entfallen.

Wie aus Figur 1 zu erkennen ist, weist die Injektionsvorrichtung, insbesondere die Antriebseinheit eine Bremse 17, 18 auf, die ein sich drehendes Teil, in diesem Beispiel das Übertragungselement 7, K2, 5 oder/und die Antriebsbewegung abbremst. Bei herkömmlichen Injektionsvorrichtungen besteht bei Fehlanwendungen, z.B. dann, wenn kein Produktbehältnis eingelegt ist und dennoch eine Betätigung der Injektinosvorrichtung vorgenommen wird, die Gefahr von Überbelastung oder sogar einer Schädigung der Bauteile der Injektionsvorrichtung. Bei einem eingelegten Produktbehältnis 27 wird durch die Viskosität des Produkts bei der Produktausschüttung eine Dämpfung der auftretenden Kräfte und Bewegungen vorgenommen. Bei einem fehlenden Produktbehältnis fehlt ein solches Dämpfungsglied. Abhilfe schafft die Bremse 17, 18, die Überbelastungen vermeidet.

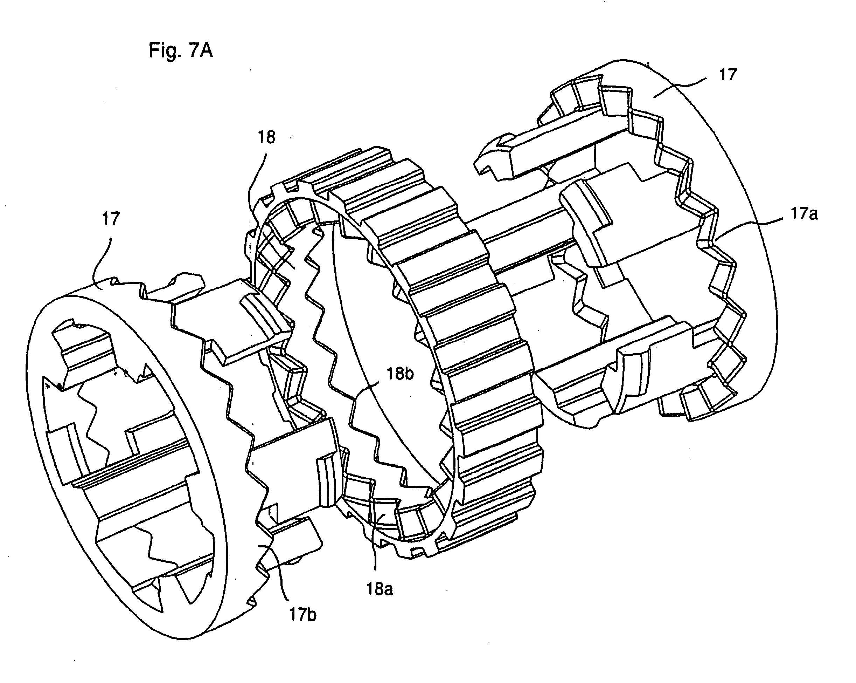

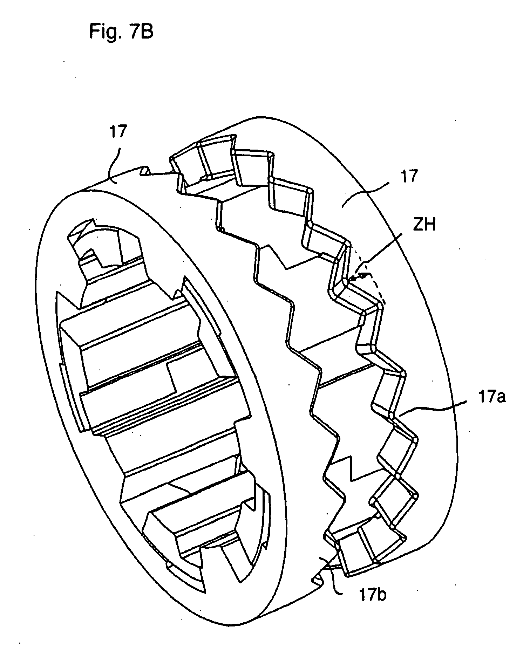

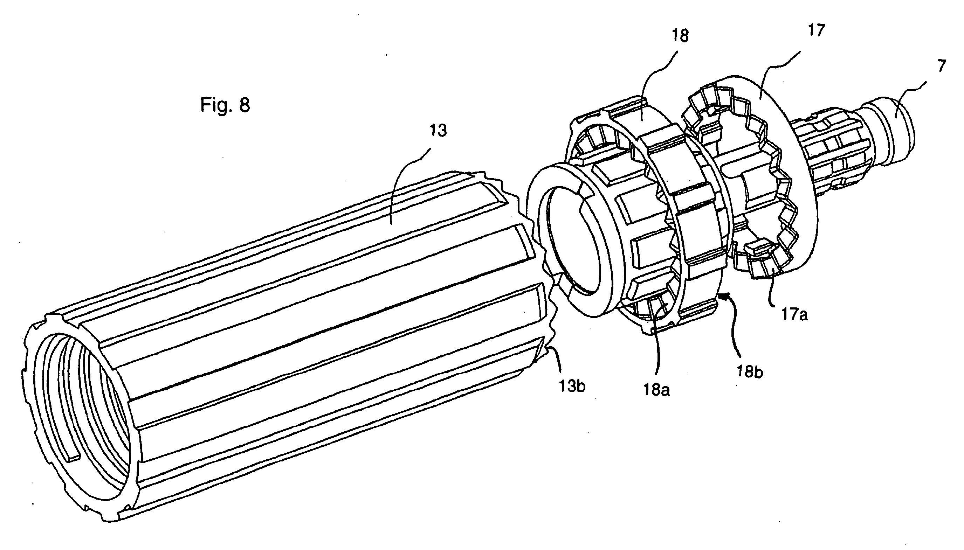

Die Figuren 7A, 7B und 8 zeigen in vergrößerter Darstellung einen für die Vorrichtung aus Figur 1 geeigneten Bremsmechanismus gemäß einer ersten und zweiten Ausführungsform, deren Wirkungsweisen ähnlich sind. Die erste Ausführungsform gemäß Figuren 7A, 7B weist zwei miteinander dreh- und vorzugsweise auch axialfest miteinander verrastete Bremsbackenhälften 17 auf, die zueinander weisende Profileabschnitte aufweisen, zwischen denen ein Ringspalt gebildet ist, in dem eine Bremsscheibe 18 aufgenommen ist. Der Ringspalt weist eine definierte Breite auf, wobei die Bremsbackenhälften alternativ relativ zu einander axial verschiebbar sein könnten. Die Bremsbacke 17 könnte einteilig ausgebildet sein. Die Bremsscheibe 18 ist relativ zu dem Gehäuse 12 drehfest und axial bewegbar aufgenommen, was durch die profilierte, in eine profilierte innere Umfangsfläche des Gehäuseteils 12b eingreifende äußere Umfangsfläche der Bremsscheibe, bewirkt wird. Zumindest eine Bremsbackenhälfte 17 oder die ganze Bremsbacke ist zumindest drehfest an dem Antriebsstrang bzw. dem Übertragungselement angeordnet. Die hülsenförmige Bremsbacke 17 weist radial nach innen weisende Abragungen auf, die in ein entsprechendes Profil der Antriebshülse 7 eingreifen. Die Bremsscheibe 18 kann sich zwischen den Bremsbackenhälften 17 bewegen. Die Bremsscheibe 18 ist verdrehgesichert z.B. durch Führung in einer Nut und axial verschiebbar in der Injektionsvorrichtung bzw. dem Gehäuseteil 12b gelagert. Die Bremsscheibe 18 ist an der Ober- und Unterseite durch stirnseitig umlaufend in beide Richtungen vorstehende Zähne 18a, 18b mit gleicher oder unterschiedlicher Zahnhöhe ZH verzahnt und zwischen der Gewindehülse 13 und der Bremsbacke 17, vorzugsweise mit einem kleinen Spiel von etwa einer Zahngröße oder Zahnhöhe ZH oder größer, gelagert oder verschiebbar eingespannt, welche entsprechende Gegenverzahnungen 13b bzw. 17a vorzugsweise mit entsprechender oder gleicher Zahnhöhe ZH besitzen.

Die Bremsbacke 17 wird durch die drehmomentfeste Verbindung zwischen Übertragungelement 7, K2, 5 z.B. bei einem Ausschüttvorgang oder beim so genannten Leerschießen, d.h. bei nicht eingelegtem Produktbehältnis, in eine Drehung Relativ zur Bremsscheibe 18 versetzt. Dabei sorgt die Anordnung der Bremsbackenverzahnungen 17a, 17b dafür, dass die Bremsscheibe 18 axial zwischen der Gewindehülse 13 und der Bremsbacke 17 oszilliert. Dadurch geraten die distale Verzahnung 18a und proximale Verzahnung 18b der Bremsscheibe 18 abwechselnd mit der entsprechenden Gegenverzahnung 17b und 17a in Kontakt. Durch die dabei auftretende Reibung, die elastische Deformation und vor allem die oszillierende Masse tritt ein entsprechender Verlust auf, wodurch die maximale Winkelgeschwindigkeit ω der sich drehenden Teile 13 und 17 begrenzt wird.

Die in Figur 8 gezeigte Ausführungsform arbeitet auf dem gleichen Prinzip, mit dem Unterschied, dass eine der zwei Bremsbackenhälften bzw. deren stimseites Zahnprofil durch das Übertragungselement oder die mit dem Übertragungselement drehfest verbundene Gewindehülse 13 gebildet wird. Es kann zwischen den Profilen 17a und 13b ein fest definierter Abstand vorgesehen sein oder aber ein variabler Abstand indem die Bremsbackenhälfte 17 relativ zu der Gewindehülse 13 axial verschiebbar ist. Durch die Feder 19 könnten die Profile 13b und 17a aufeinander zu gerückt werden, so dass sie in den Eingriff mit den Profilen 18a und 18b geraten.

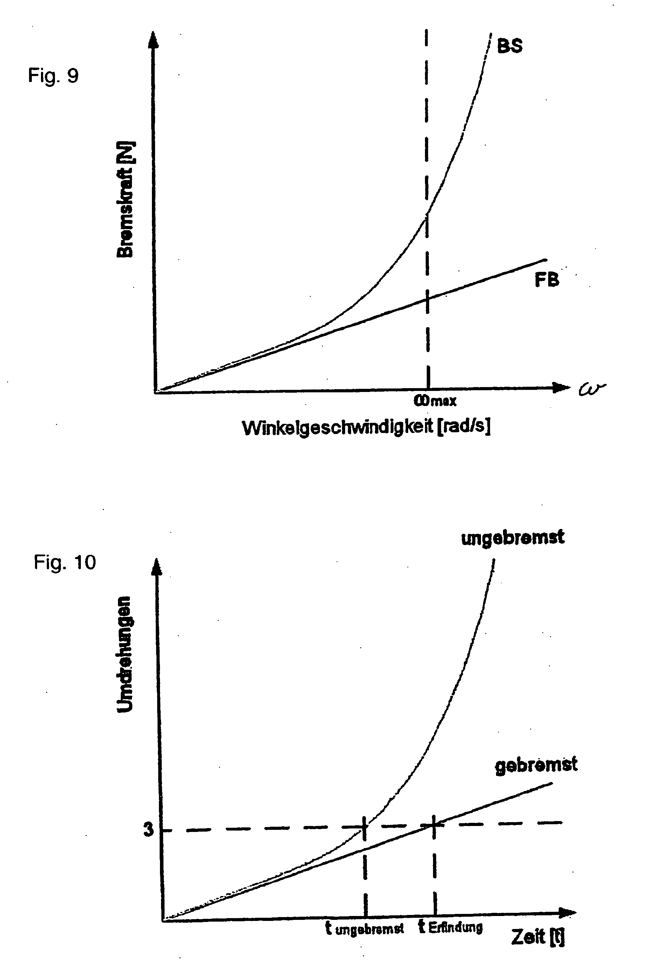

Durch die mit der Winkelgeschwindigkeit ω zunehmende Vibration oder Schwingung der Bremsscheibe 18 zwischen der Gewindehülse 13 und der Bremsbacke 18 nimmt die Bremskraft bei zunehmender Winkelgeschwindigkeit ω überproportional zu, so dass der in Figur 9 schematisch gezeichnete Verlauf BS der Bremskraft realisiert werden kann.

Figur 9 zeigt schematisch den Verlauf der mit der erfindungsgemäßen Bremsvorrichtung erzielbaren Bremskraft, wobei gesehen werden kann, dass die Bremskraft zunehmend mit der Winkel- oder Drehgeschwindigkeit ω ansteigt. Idealerweise ist die Bremskraft bis zur maximal zulässigen Winkelgeschwindigkeit ωₘₐₓ relativ gering oder Null und steigt bei der maximal zulässigen Winkelgeschwindigkeit ωₘₐₓ stark an.

Figur 10 zeigt den über die Zeit aufgetragenen Drehwinkel der Anzeigetrommel 4, welche im Ausführungsbeispiel drei vollständige Umdrehungen (3x360°) durchlaufen kann. Dabei kann aus Figur 10 gesehen werden, dass die Anzeigetrommel 4 drei vollständige Umdrehungen nach der Zeit t_{ungebremst} durchlaufen hat, welche kürzer ist, als die Zeit t_{Erfindung} im Falle einer gebremsten Drehbewegung der Anzeigetrommel 4, bei welcher sich der Drehwinkel im Idealfall linear in Abhängigkeit von der Zeit erhöht.

Durch die mittels der oszillierenden Bremsscheibe 18 erzeugten Bremskraft kann die maximal mögliche Winkelgeschwindigkeit ωₘₐₓ einer Ausschüttbewegung verringert oder begrenzt werden, so dass die sich zurückdrehende Anzeigetrommel 4 nur mit einer durch die Bremse 13, 17, 18 vorgegebenen maximalen Geschwindigkeit an in Umfangsrichtung wirkenden Anschlag oder das Gehäuseteil 12b anschlagen kann. Ist die Bremse 13, 17, 18 geeignet ausgelegt, ist die maximal mögliche Anschlaggeschwindigkeit der Anzeigetrommel 4 so gering, dass durch den Anschlagimpuls keine nennenswerten Verformungen oder Beschädigungen auftreten können. Alternativ zur Ausbildung der Bremse durch eine zwischen der Gewindehülse 13 und der Bremsbacke 17 oszillierende Bremsscheibe 18 können auch andere Bremsmechanismen verwendet werden.

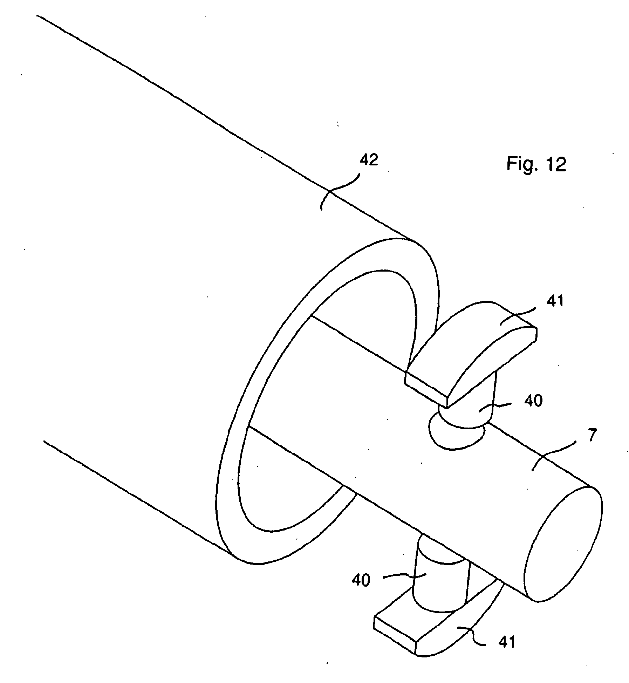

Beispielsweise kann die Bremse alternativ ergänzend oder auch gemäß einer dritten Ausführungsform durch eine in Figur 12 gezeigte Fliehkraftbremse realisiert werden, wobei z.B. an dem Übertragungselement 7, K2, 5 oder/und der Antriebswelle 7 und/oder einem anderen sich mit der Antriebswelle 7 drehenden Teil, wie z.B. dem Kupplungsglied 10, der Gewindehülse 13 oder der Anzeigetrommel 4, z.B. nach außen bewegbare Bremsbacken 41 angebracht sind, die eine Masse aufweisen und die die Drehung des rotierenden Teils mitmachen. Die Bremsbacken 41 können aber müssen nicht mittels einer Feder nach innen oder nach außen vorgespannt sein. Die Bremsbacken können durch die Fliehkraft verschwenkbar oder radial nach außen bewegbar sein, um in einen Bremseingriff mit einer Hülse 42, wie z.B. dem Gehäuse 12, kommen zu können. In der dritten Ausführungsform sind radial nach außen ragende Stifte 40 oder Befestigungen angebracht, an deren Enden sich z.B. mit der Feder vorgespannte Bremsbacken 41 befinden. Ist die Drehgeschwindigkeit des sich ungebremst oder nur teilweise gebremst drehenden Elements 4, 7, 10 und/oder 13 ausreichend groß, werden die Bremsbacken 41 durch die Zentrifugalkraft, ggf. auch zusätzlich durch Federunterstützung, radial nach außen bewegt und können z.B. an einer äußeren stehenden Hülse 42 in Anlage kommen und durch eine Reibung für die gewünschte Bremswirkung sorgen. Die äußere stehende Hülse kann z.B. auch durch das Gehäuse 12 oder das Gehäuseteil 12b gebildet werden.

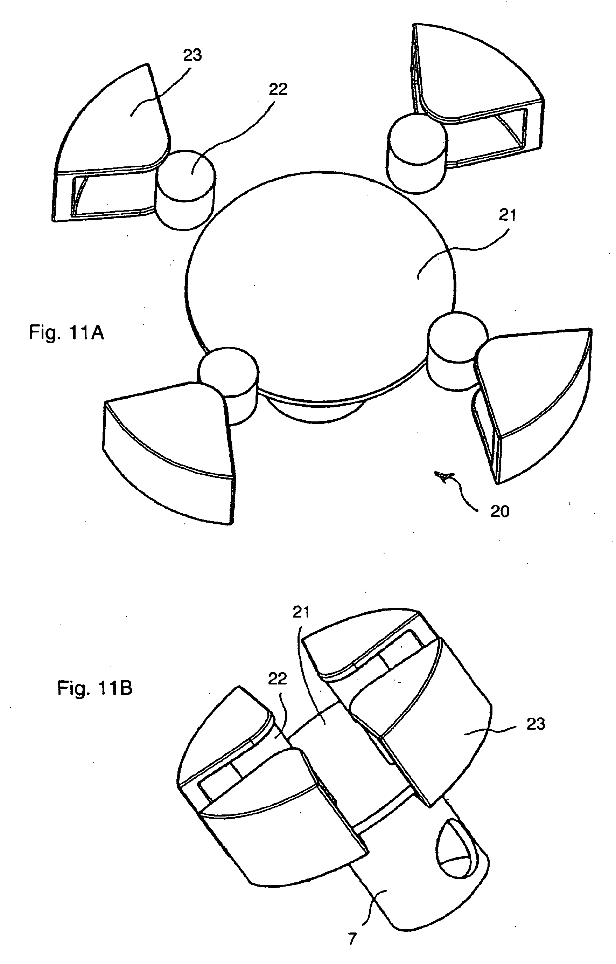

Bei einer vierten, in den Figuren 11A und 11B gezeigten Ausführungsform kann die Bremse als Wirbelstrombremse 20 ausgebildet werden, wobei eine Bremsscheibe 21 mit einem sich drehenden und zu bremsenden Teil, wie z.B. dem Übertragungselement 7, K2, 5, der Antriebswelle 7, der Gewindehülse 13 oder der Anzeigetrommel 4, und die mit der Bremsscheibe wechselwirkenden Elemente mit dem Gehäuse oder einem gehäusefesten Element oder mit einem Element relativ zu dem sich die Bremsscheibe dreht, verbunden sein können

Die Bremsscheibe 21 ist vorzugsweise aus einem sehr guten elektrischen Leiter gefertigt, wie z.B. Reinaluminium oder Kupfer. Als Material für die axial magnetisierten Magnete 22 können Seltenerdelegierungen, wie z.B. Neodym, verwendet werden. Das Permanentmagnetfeld kann mittels eines Magnetjochs 23 aus Eisen zum Luftspalt gelenkt werden, wo es möglichst senkrecht die Bremsscheibe 21 durchdringt. Die Bremskraft wird durch die Fläche und die Flussdichte im Luftspalt und den indizierte Strom in der Bremsscheibe 21 beeinflusst, wobei die Fläche möglichst groß, der Luftspalt möglichst klein und die Scheibenstärke möglichst groß sein sollte. Das Bremsmoment entsteht über den gemittelten Radius (Wirkradius). Es können Bremsen mit mehreren Magnetsystemen, welche auf eine Scheibe 21 wirken, ausgelegt werden.

Zur Berechnung der Stromdichte, der Bremsleistung und damit des Bremsmoments einer Wirbelstrombremse werden gewöhnlich Näherungsrechnungen verwendet. Unter Vernachlässigung des Effekts durch den Luftspalt wird ein einheitlicher zylindrischer magnetischer Fluss angenommen und vorausgesetzt, dass der Poldurchmesser gegenüber dem Radius der Scheibe 21 genügend klein ist. Bei hohen Drehzahlen wird die Näherung ungenau, da unter anderem die Magnetfelder, welche durch die Wirbelströme verursacht werden, zu einer nicht vernachlässigbaren Rückwirkung und damit zu Unlinearität führen.

Die Magnete 22 und das Magnetjoch 23 sind vorzugsweise mit dem Gehäuse 12 der Injektionsvorrichtung oder dem Gehäuseteil 12b oder einem anderen sich nicht drehenden Teil verbunden, um die gewünschte Wirbelstrombremswirkung der Bremsscheibe 21 erzeugen zu können.

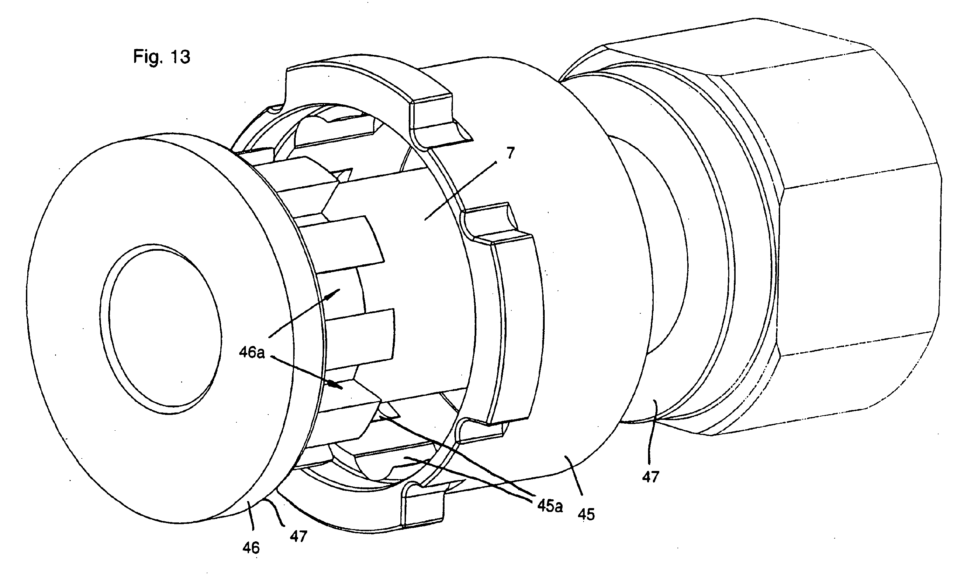

Gemäß einer vierten, in den Figuren 13 und 14 dargestellten Ausführungsform kann die Bremse als Fluidbremse ausgebildet werden. Wird ein normales Fluid als Bremsmedium verwendet, so wird die in Figur 4 für die Wirbelstrombremse gezeigte lineare Bremskennlinie FB erhalten. Soll jedoch eine in Abhängigkeit von der Winkelgeschwindigkeit ω stärker ansteigende Bremskraft realisiert werden, können so genannte nichtnewtonsche Fluide verwendet werden, bei welchen im Gegensatz zum newtonschen Fluid die Viskosität nicht konstant bleibt, sondern ansteigt, wenn sich eine auf das Fluid einwirkende Scherkraft erhöht, was bei zunehmender Geschwindigkeit der Fall ist. Es handelt sich dabei um so genannte anomalviskose Fluide.

Bei der Fluidbremse wird die Bremskraft über zwei sich gegeneinander bewegende Fluidoberflächen erzeugt. Insbesondere wird die Bremskraft über ein Fluidvolumen erzeugt, das durch eine Relativbewegung abgeschert wird. Die bei solchen Bewegungen auftretenden Scherspannungen entsprechen der Bremskraft. Das Volumen wird von einer zweigeteilten Kammer 45a, 46a gebildet, in der das Fluid angeordnet ist. Ein Kammerteil 46a ist in einem sich drehenden Teil 46 und die andere Kammer 45a in einem Teil 45 angeordnet relativ zu dem sich das Teil 46 drehen kann. Das Teil 46 kann z.B. mit der Antriebswelle7 oder dem Übertragungselement 7, K2, 5 oder einem anderen sich bei einer Produktausschüttung drehenden Teil drehfest verbunden sein. Das Teil 45 ist zumindest drehfest mit dem Gehäuse 12 oder einem gehäusefesten Teil verbunden. Zudem kann das Teil 45 relativ zu dem Gehäuse 12 axial bewegbar oder axialfest sein. Das hülsenförmige Teil 45 kann als Bremsengehäuse und das in der Hülse 45 gelagerte Teil 46 kann als Bremsenwelle bezeichnet werden. Im zusammengesetzten Zustand der Bremse sind die über den Außenumfang der Bremsenwelle verteilten Fluidkammerhälften 46a axial auf Höhe der über den Innenumfang des Bremsengehäuses verteilten Fluidkammerhälften 45a. Es können mehr, gleich viele oder weniger Fluidkammerhälften 45a als 46a vorgesehen sein. Zwischen dem Innendurchmesser des Bremsengehäuses 45 und dem Außendurchmesser der Bremsenwelle 46 jeweils im Bereich der Fluidkammerhälften 45a, 46a, ist im zusammengesetzten Zustand ein dünner Spalt, der so bemessen sein kann, dass Fluid in den Spalt transportiert wird oder dass kein oder so gut wie kein Fluid in den Spalt transportiert wird, wenn sich die Bremsenwelle 46 relativ zu dem Bremsengehäuse 45 dreht. Axial kann das Bremsengehäuse 45 auf beiden Stirnseiten mit gleitfähigen Dichtelementen 47 abgedichtet sein, so dass kein Fluid aus der Bremse austreten kann. Die Dichtelemente 47 können von einem Deckel gebildet werden. Als Deckel kann z.B. ein separates Teil vorgesehen sein oder die Kupplungswelle dienen.

Vorteilhaft an der erfindungsgemäßen Lösung ist, dass die Bremskraft mit zunehmender Winkelgeschwindigkeit ansteigt, d.h. eine durch z.B. die Viskosität des Produkts bei der Produktausschüttung gedämpfte Bewegung kann ungebremst oder fast ungebremst stattfinden. Die Bremskraft der erfindungsgemäßen Bremse nimmt vorzugsweise erst bei den oben beschriebenen Problemfällen, bei welchen hohe Drehgeschwindigkeiten auftreten können, auf ein bedeutendes Maß zu.

### Bezugszeichenliste:

| | | | |
|---|---|---|---|
| 1 | Flansch | 31 | Kappe |
| 2 | Abtriebsglied / Kolbenstange | 38 | Federglied |
| 3 | Antriebsfeder / Spiralfeder | 40 | Radialführung |
| 4 | Anzeigetrommel | 41 | Bremsbacke |
| 5 | Kupplungshülse | 42 | Bremshülse |
| 6 | Eingriffsglied / Gewindeeinsatz | 45 | Bremsengehäuse |
| 7 | Antriebsglied / Antriebswelle | 46 | Bremsenwelle |
| 8 | Federhülse | 47 | Dichtelement |
| 9 | Dosierelement / Dosierknopf | 50 | Bajonetthülse |
| 10 | Kupplungselement | K1 | erste Kupplung |
| 11 | Ratschenfeder | K2 | zweite Kupplung |
| 12 | Gehäuse | K3 | dritte Kupplung |
| 13 | Übertragungsglied / Gewindehülse | | |
| 14 | Kupplungselement / Arretierhülse | | |
| 15 | Betätigungselement / Druckknopf | | |
| 16 | Produktbehältnisaufnahme | | |
| 17 | erstes Bremselement | | |
| 18 | zweites Bremselement | | |
| 19 | Federelement / Wendelfeder | | |
| 20 | Wirbelstrombremse | | |
| 21 | Bremsscheibe | | |
| 22 | Magnet | | |
| 23 | Magnetjoch | | |
| 24 | Stützring | | |
| 25 | Halteglied | | |
| 26 | Eingriffsglied / Führungshülse | | |
| 27 | Produktbehältnis | | |
| 28 | Kolben | | |
| 29 | Lager / Kugellager | | |
| 30 | Läufer | | |

## Patentansprüche

1. Bremsvorrichtung für eine Injektionsvorrichtung zur Erzeugung einer Bremswirkung auf ein sich bewegendes oder drehendes Teil (2,4, 7,13,17) der Injektionsvorrichtung
**dadurch gekennzeichnet, dass**
die Bremsvorrichtung eine Bremsscheibe (18) aufweist, welche im Zusammenwirken mit mindestens einem Gegenelement (13,17) eine Bremswirkung erzeugt, welche die Bewegung oder Drehung des sich bewegenden oder drehenden Teils (2,4, 7, 13,17) in Abhängigkeit von der Geschwindigkeit des sich bewegenden oder drehenden Teils dämpft, wobei die Dämpfung bei einer grossen Geschwindigkeit gross ist und bei einer kleinen Geschwindigkeit klein ist.

2. Bremsvorrichtung nach Anspruch 1, wobei das sich drehende Teil eine Gewindestange(2), eine Hülse, eine Anzeigetrommel (4), eine Antriebswelle (7), eine Gewindehülse(13), eine Bremsbacke (17) oder ein damit verbundenes oder gekoppeltes Teil ist.

3. Bremsvorrichtung nach einem der vorhergehenden Ansprüche, wobei die Bremsscheibe(18) in Verbindung mit mindestens einem Gegenelement (13, 17) als Reibungsbremse wirkt.

4. Bremsvorrichtung nach einem der vorhergehenden Ansprüche, wobei die Bremsscheibe (18) mindestens eine stimseitig auf einer Seite oder auf beiden Seiten angebrachte zum Teil oder vollständig umlaufende Profilierung oder Verzahnung(18a, 18b) aufweist.

5. Bremsvorrichtung nach dem vorhergehenden Anspruch, wobei mindestens ein Gegenelement (13, 17) der Bremsscheibe (18) eine korrespondierende oder Gegenverzahnung (13b, 17a) oder ein Gegenprofil aufweist.

6. Bremsvorrichtung nach dem vorhergehenden Anspruch, wobei die Gegenelemente(1 3, 17) fest miteinander verbunden sind und die Gegenprofile oder Gegenverzahnungen (13b, 17a) einen Abstand voneinander aufweisen, welcher gleich oder größer ist als eine Profiltiefe oder Zahnhöhe (ZR) einer oder beider Verzahnunger (18a, 18b) der Bremsscheibe (18).

7. Bremsvorrichtung nach einem der vorhergehenden Ansprüche, wobei die Bremsscheibe(18) drehfest und axial verschiebbar und mindestens ein Gegenelement (13, 17) drehbar angeordnet ist.

8. Injektionsvorrichtung mit einer Bremsvorrichtung nach einem der vorhergehenden Ansprüche.

## Claims

1. A brake device for an injection device for producing a braking action on a moving or rotating part (2, 4, 7, 13, 17) of the injection device,
**characterised in that**
the brake device has a brake disc (18) which co-operating which at least one counterpart element (13, 17) produces a braking action which damps the movement or rotation of the moving or rotating part (2, 4, 7, 13, 17) in dependence on the speed of the moving or rotating part, wherein the damping is great with a high speed and slight with a low speed.

2. A brake device according to claim 1 wherein the rotating part is a threaded rod (2), a sleeve, a display drum (4), a drive shaft (7), a threaded sleeve (13), a brake shoe (17) or a part connected or coupled thereto.

3. A brake device according to one of the preceding claims wherein the brake disc (18) in conjunction with at least one counterpart element (13, 17) acts as a friction brake.

4. A brake device according to one of the preceding claims wherein the brake disc (18) has at least one profiling or tooth configuration (18a, 18b) which is disposed in frontal relationship on one or both sides and which extends partly or completely therearound.

5. A brake device according to one of the preceding claims wherein at least one counterpart element (13, 17) of the brake disc (18) has a corresponding or counterpart tooth configuration (13b, 17a) or a counterpart profile.

6. A brake device according to the preceding claim wherein the counterpart elements (13, 17) are fixedly connected together and the counterpart profiles or counterpart tooth configurations (13b, 17a) are at a spacing from each other which is equal to or greater than a profile depth or tooth height (ZR) of one or both of the tooth configurations (18a, 18b) of the brake disc (18).

7. A brake device according to one of the preceding claims wherein the brake disc (18) is arranged non-rotatably and axially displaceably and at least one counterpart element (13, 17) is arranged rotatably.

8. An injection device comprising a brake device according to one of the preceding claims.

## Revendications

1. Dispositif de freinage pour un dispositif d'injection destiné à générer une action de freinage sur une pièce (2, 4, 7, 13, 17) rotative ou mobile du dispositif d'injection
**caractérisé en ce que**
le dispositif de freinage présente un disque de frein (18) qui génère une action de freinage en coopération avec au moins un élément antagoniste (13, 17), laquelle action de freinage amortit le mouvement ou la rotation de la pièce rotative ou mobile (2, 4, 7, 13, 17) en fonction de la vitesse de cette dernière, l'amortissement étant important en cas de grande vitesse et moindre en cas de petite vitesse.

2. Dispositif de freinage selon la revendication 1, la pièce rotative étant une tige filetée (2), une douille, un tambour d'affichage (4), un arbre d'entraînement (7), une douille filetée (13), un segment de frein (17) ou une pièce couplée ou reliée à celle-ci.

3. Dispositif de freinage selon l'une quelconque des revendications précédentes, le disque de frein (18) agissant comme un frein de friction en liaison avec au moins un élément antagoniste (13, 17).

4. Dispositif de freinage selon l'une quelconque des revendications précédentes, le disque de frein (18) présentant au moins un profilage ou une denture (18a, 18b) tournant en partie ou complètement, montée sur l'avant, sur un côté ou les deux côtés.

5. Dispositif de freinage selon la revendication précédente, au moins un élément antagoniste (13, 17) du disque de frein (18) présentant une denture correspondante ou antagoniste (13b, 17a) ou un profilé antagoniste.

6. Dispositif de freinage selon la revendication précédente, les éléments antagonistes (13, 17) étant solidement reliés entre eux et les profilés antagonistes ou dentures antagonistes (13b, 17a) présentant un écartement les unes des autres qui est égal ou supérieur à une profondeur de profilé ou hauteur de dent (ZR) d'une ou de deux dentures (18a, 18b) du disque de frein (18).

7. Dispositif de freinage selon l'une quelconque des revendications précédentes, le disque de frein (18) étant disposé de manière solidaire en rotation et axialement mobile et au moins un élément antagoniste (13, 17) étant disposé de manière rotative.

8. Dispositif d'injection avec un dispositif de freinage selon l'une quelconque des revendications précédentes.
